(19) Europäisches Patentamt
European Patent Office
Office européen des brevets

(11) **EP 3 481 871 B1**

(12) **FASCICULE DE BREVET EUROPEEN**

(45) Date de publication et mention de la délivrance du brevet:
**21.08.2024 Bulletin 2024/34**

(21) Numéro de dépôt: **17732528.9**

(22) Date de dépôt: **09.06.2017**

(51) Classification Internationale des Brevets (IPC):
*C08B 30/18* (2006.01)    *C08L 3/02* (2006.01)

(52) Classification Coopérative des Brevets (CPC):
**C08B 30/18; C08L 3/02**

(86) Numéro de dépôt international:
**PCT/FR2017/051462**

(87) Numéro de publication internationale:
**WO 2018/007697 (11.01.2018 Gazette 2018/02)**

(54) **COMPOSITION DE POLYMÈRE DE GLUCOSE HYDROGÉNÉE CONTENANT DES FIBRES ALIMENTAIRES**

HYDRIERTE GLUCOSEPOLYMERZUSAMMENSETZUNG MIT BALLASTSTOFFEN

HYDROGENATED GLUCOSE POLYMER COMPOSITION CONTAINING DIETARY FIBRES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.07.2016 FR 1656604**
**28.02.2017 FR 1751618**

(43) Date de publication de la demande:
**15.05.2019 Bulletin 2019/20**

(73) Titulaire: **Roquette Frères**
**62136 Lestrem (FR)**

(72) Inventeurs:
• **IBERT, Mathias**
**59930 La Chapelle d'Armentieres (FR)**
• **BOIT, Baptiste**
**59253 La Gorgue (FR)**

(74) Mandataire: **Plasseraud IP**
**104 Rue de Richelieu**
**CS92104**
**75080 Paris Cedex 02 (FR)**

(56) Documents cités:
**EP-A1- 0 654 483       EP-A2- 0 516 491**
**EP-A2- 0 561 088       WO-A1-92/14761**
**WO-A1-99/36442        FR-A1- 2 668 147**
**FR-A1- 2 764 294       US-A- 5 424 418**
**US-A1- 2003 096 055**

EP 3 481 871 B1

## Description

### Objet de l'invention

L'invention porte sur une nouvelle composition de polymères de glucose hydrogénée contenant des fibres alimentaires et comprenant une très faible teneur en sucres réducteurs. Un autre objet de l'invention porte sur un procédé de fabrication de cette composition, ledit procédé comprenant une combinaison d'étapes spécifiques d'hydrogénation et d'alcalinisation permettant de réduire très fortement la quantité des fonctions réductrices du polymère de glucose, tout en ne dégradant que peu leur structure. L'invention a également pour objet un produit alimentaire ou pharmaceutique comprenant ladite composition.

### Art antérieur

Les polymères de glucose sont couramment utilisés dans de nombreuses industries, telles que les industries alimentaires ou pharmaceutiques.

Les polymères de glucose les plus courants sont les hydrolysats d'amidon. Les hydrolysats d'amidon sont généralement regroupés sous deux grandes familles, les maltodextrines et les sirops de glucose. L'appartenance à chacune de ces familles est déterminée selon leur Dextrose Equivalent (DE), le DE étant le rapport du nombre de sucres réducteurs sur le nombre de sucres totaux multiplié par 100. Les maltodextrines présentent un DE inférieur à 20 et les sirops de glucose présentent un DE de 20 ou plus.

Un hydrolysat d'amidon est essentiellement digestible et ne comprend pas de fibres alimentaires.

On entend par « fibres alimentaires » selon la présente invention des structures polymères de glucose n'étant pas ou peu digérées par l'Homme.

Il existe d'autres polymères de glucose contenant des fibres alimentaires non digestibles. Si les amidons et les hydrolysats d'amidon possèdent uniquement des liaisons glucidiques de type $\alpha$-1,4 et $\alpha$-1,6, les polymères de glucose contenant des fibres alimentaires non digestibles comprennent des liaisons atypiques de type 1,2 (en anoméries $\alpha$ ou $\beta$) et 1,3 (en anoméries $\alpha$ ou $\beta$) ou encore des liaisons atypiques de type $\beta$-1,4 et $\beta$-1,6. Or, l'ensemble de ces liaisons atypiques ne sont pas ou difficilement hydrolysées par les enzymes de digestion humaines.

Ces polymères de glucose contenant des fibres alimentaires sont généralement fabriqués par un traitement thermique d'un amidon ou d'un hydrolysat d'amidon, l'hydrolysat utile à la fabrication pouvant notamment être ou comprendre du glucose et/ou du maltose. Ce traitement thermique est réalisé en utilisant une catalyse acide ou enzymatique ; il permet la formation des liaisons atypiques précitées et il en résulte la formation de polymères de glucose contenant des fibres alimentaires.

Les polymères de glucose précités comprennent des sucres réducteurs terminaux, qui sont les fonctions les plus réactives du polymère de glucose. De manière générale, on considère que le sucre réducteur est le principal responsable de l'instabilité thermique des polymères. C'est notamment ce sucre réducteur qui est susceptible de réagir avec des espèces azotées et conduire à des réactions de Maillard.

C'est ainsi que, pour rendre les polymères de glucose plus stables thermiquement, il est connu d'hydrogéner la fonction réductrice des hydrolysats d'amidon, comme décrit dans la demande WO 99/36442 qui décrit plus particulièrement un procédé d'hydrogénation d'un malto-oligosaccharide, et ceci dans des conditions suffisamment « douces » pour ne pas dégrader le malto-oligosaccharide. Cette réaction est préférentiellement réalisée à l'aide d'un catalyseur de type nickel activé. Avantageusement, l'étape d'hydrogénation est réalisée à une température allant de 110 à 120°C et une pression allant de 400 à 700 psi. Ce document conseille également de conduire la réaction d'hydrogénation à un pH allant de 5,0 à 6,0. Le malto-oligosaccharide résultant présente un DE « essentiellement zéro », c'est-à-dire selon ce document un DE d'au plus 1. Le polymère de glucose ne contient pas de fibres alimentaires et la teneur en sucres réducteurs n'est pas indiquée.

Par ailleurs, comme la préparation de polymères de glucose contenant des fibres alimentaires comprend une étape de traitement thermique, ces polymères présentent généralement, contrairement aux hydrolysats d'amidons décrits précédemment, un problème de coloration. Ce problème de coloration peut être plus important lorsque le traitement thermique est fait à haute température, par exemple à une température dépassant 100°C. Les phénomènes de coloration sont classiquement attribués à la présence de liaisons insaturées. Ces liaisons insaturées peuvent être celles des glucoses réducteurs ou des liaisons insaturées internes au polymère (par exemple alcènes) formées lors de l'étape de traitement thermique.

Ce problème de coloration des polymères de glucose contenant des fibres alimentaires peut être limité soit en utilisant des conditions de préparation douces comme décrit dans le document EP 530.111, soit en conduisant une purification sur résines cationiques et anioniques ou avec du noir de carbone comme décrit dans les documents EP 485.304 et EP 516.491. Une autre solution est décrite dans le brevet US 4.622.233 dans lequel la décoloration du polymère de glucose est réalisée par des peroxydes ou des chlorites.

Une autre solution pour décolorer le produit consiste à hydrogéner le polymère de glucose contenant des fibres alimentaires.

Cette hydrogénation a notamment été décrite dans la demande de brevet WO 92/14761. Le polydextrose hydrogéné présente un DE d'« essentiellement zéro », c'est-à-dire selon ce document un DE inférieur à 1. Ce document décrit l'hydrogénation d'un polydextrose dans une étape consistant à chauffer en présence d'un catalyseur d'hydrogénation et de dihydrogène (dans la suite de la description « hydrogène »). Dans les conditions de l'Exemple 9, le polydextrose est mis sous forme d'une solution à 40% de matière sèche, de pH égal à 6,2 et cette solution comprenant en outre du nickel de Raney est portée à 140-150°C sous une pression d'hydrogène de 1400 Psig pendant 90 minutes. Le polydextrose hydrogéné obtenu présente une quantité de sucres réducteurs de 0,2%. Ce document décrit également une hydrogénation en utilisant un agent réducteur un borohydrure. Dans les conditions de l'Exemple 20 où la quantité de sucres réducteurs a été déterminée, cette dernière est de 0,4%. Ces polymères de glucose hydrogénés sont présentés comme présentant une couleur et un goût améliorés, tout en étant moins réactifs avec d'autres produits comprenant des fonctionnalités amines.

Le brevet US 5,424,418 décrit de la même manière un procédé de fabrication d'un polydextrose hydrogéné comprenant l'hydrogénation d'un polydextrose pour former un polydextrose hydrogéné suivie d'une étape de chromatographie de ce polydextrose hydrogéné pour en éliminer le sorbitol. Cette étape d'hydrogénation est réalisée sans changement de pH (pH 7 ou 8). Tout préférentiellement, la quantité de sucres réducteurs dans le produit obtenu est inférieure à 0,15%. Toutefois, les exemples démontrent qu'il n'est pas possible d'obtenir avec le procédé de ce document une composition de polymère hydrogéné comprenant une quantité de sucres réducteurs très faible. Même avant l'étape de chromatographie, le polydextrose hydrogéné comprend une quantité de sucres réducteurs élevée (dans les exemples 0,50%, 0,45% et, au minimum, 0,12%) et l'étape de chromatographie subséquente ne permet pas de réduire cette quantité de sucres réducteurs. En effet, le sorbitol qui est éliminé de la composition lors de l'étape de chromatographie n'est pas un sucre réducteur.

Le document EP 654483 au nom de la Demanderesse décrit quant à lui, selon une variante, un procédé dans lequel est réalisée une étape d'hydrogénation à un pH compris entre 4 et 8. Plus particulièrement, à l'exemple 5 est décrite l'hydrogénation d'un polydextrose ayant subi préalablement un traitement enzymatique et à la glucose oxydase. Selon l'exemple 5, cette hydrogénation est réalisée en présence de nickel de Raney sur une solution à 40% de matière sèche de pH égal à 7, sous pression de 50 bars d'hydrogène. Le polydextrose obtenu présente une quantité de sucres réducteurs de 0,10%, ceci malgré l'utilisation préalable de glucose oxydase, qui permet également de réduire la quantité de sucres réducteurs dans la composition. L'hydrogénation est réalisée sans modification de pH au cours de la réaction.

Le document EP 368451 qui décrit également aux exemples 2 et 10 l'hydrogénation d'une dextrine difficilement digestible, qui est de plus haute masse moléculaire que le polydextrose. Cette hydrogénation est réalisée en présence de nickel de Raney, à 130°C pendant 90 minutes ou 2 heures, sur une suspension aqueuse de dextrine à 40% de matière sèche dont le pH est réglé à 9,5, avec une pression d'hydrogène de 95 bar. La quantité de sucres réducteurs n'est pas déterminée mais la Demanderesse a pu constater qu'en suivant cet enseignement, la quantité de sucres réducteurs était bien supérieure à 0,1% : elle est d'au moins 0,25% et cette hydrogénation conduit à une dégradation de la dextrine difficilement digestible.

Le document US 2003/0096055 A1 décrit des dérivés hydrogénés solubles d'amidon contenant des fibres qui présentent une couleur améliorée et une teneur en sucres réducteurs réduite d'au maximum 30% par rapport au dérivé soluble d'amidon avant hydrogénation. L'échantillon présentant la quantité de sucres réducteurs la plus faible (0,6%) n'est pas selon l'invention puisqu'elle est trop faible pour répondre aux critères revendiqués (réduction d'environ 75%).

Le document EP 516491 A2 décrit la fabrication d'un hydrolysat de pyrodexrine comprenant plusieurs étapes de traitement enzymatique et de passage en autoclave d'une pyrodextrine.

Le document FR 2 668 147 A1 décrit quant à lui un procédé de stabilisation d'un hydrolysat oxydé de polysaccharide.

Les polymères de glucose, qu'ils comprennent ou non des fibres alimentaires, sont plus ou moins difficiles à hydrogéner. En effet, quel que soit le procédé utilisé, la Demanderesse a pu observer la tendance suivante : plus la viscosité du polymère de glucose contenant des fibres alimentaires est importante, plus ce polymère est difficile à hydrogéner. La viscosité du polymère de glucose étant principalement liée à sa masse moléculaire, plus le polymère de glucose présente une masse moléculaire élevée, plus il est difficile de réduire la quantité de sucres réducteurs dans la composition. C'est ainsi que, jusqu'à ce jour, il n'a jamais été possible d'obtenir par les procédés d'hydrogénation une composition de polymère de glucose hydrogénée comprenant une quantité de sucres réducteurs en poids sec inférieure à 1000 ppm.

EP 0 561 088 A2 divulgue une composition de polymère de glucose hydrogéné présentant une quantité de sucres réducteurs de 300 ppm et un taux de polysaccharides non hydrolysables par l'amyloglucosidase, déterminé selon le Test F, de 5,0%.

Les produits Litesse® Ultra commercialisé par la société Danisco ou Fibersol® 2H commercialisé par la société Matsutani, qui sont tous les deux des polymères de glucose contenant des fibres alimentaires hydrogénés comprennent quant à eux (voir partie exemples) respectivement 0,15% et 0,25% de sucres réducteurs.

Il découle de ce qui précède que les procédés d'hydrogénation de l'art antérieur ne permettent pas d'obtenir des polymères de glucose hydrogénés qui comprennent une très faible quantité de sucres réducteurs. Or, même lorsque la quantité de sucres réducteurs est aussi faible que 0,15%, voire 0,10%, une dégradation peut se produire sous l'action de la chaleur, notamment lorsque le polymère de glucose hydrogéné est mis en présence de composés porteurs de fonctions amines, cette dégradation conduisant à la coloration du produit. Or, ces polymères étant notamment destinés aux industries alimentaires, il peut être nécessaire de pouvoir les utiliser dans toutes les conditions de température, sans provoquer de brunissement excessif du produit alimentaire les comprenant. Les produits alimentaires présentant un pH allant très généralement de 2 à 7, il serait intéressant de pouvoir fournir à l'industrie alimentaire des polymères de glucose présentant une stabilité thermique améliorée à pH acide et neutre. Dans des applications pharmaceutiques, il peut être nécessaire que ces polymères soient le moins réactifs possible avec d'autres constituants d'une préparation pharmaceutique.

Il existe donc un besoin d'obtenir de nouvelles compositions de polymères de glucose hydrogénés contenant des fibres alimentaires qui présentent une quantité de sucres réducteurs encore plus faible que celle des compositions déjà connues, notamment des polymères de glucose hydrogénés de masse moléculaire élevée et contenant des fibres alimentaires.

C'est justement l'objet de la présente invention qui va être décrite ci-après.

**Résumé de l'invention**

L'invention porte sur une composition de polymère de glucose hydrogénée présentant une teneur totale de fibres, déterminée selon la méthode AOAC 2001.03, supérieure à 50% et une quantité de sucres réducteurs en poids sec SR inférieure à 800 ppm.

La Demanderesse a en effet trouvé qu'il était tout à fait possible d'obtenir une composition de polymère de glucose hydrogénée qui présente une quantité de sucres réducteurs jamais atteinte jusqu'à ce jour, tout en ne dégradant que peu le polymère de glucose et en ne diminuant pas ou peu la teneur totale de fibres dans le polymère de glucose. La Demanderesse y est parvenue même lorsque la masse moléculaire de ce polymère de glucose est élevée. Cette composition présente de nombreux avantages et notamment une faible couleur, une faible odeur, un faible goût ainsi qu'une excellente stabilité à la température, notamment de pH acide à neutre. Ceci lui permet d'être avantageusement utilisé dans des produits alimentaires et pharmaceutiques.

C'est après de nombreux travaux que la Demanderesse a pu constater qu'un procédé particulier, comprenant une première étape d'hydrogénation d'un polymère de glucose et une seconde étape d'alcalinisation, permettait d'obtenir la composition de polymère de glucose hydrogénée de l'invention.

L'invention a donc également pour objet un procédé de fabrication d'une composition de polymère de glucose hydrogénée selon l'invention comprenant :

- une étape de fourniture d'une solution aqueuse de polymère de glucose contenant des fibres alimentaires ;
- une étape de mise en contact de la solution aqueuse avec un catalyseur d'hydrogénation pour fournir, éventuellement après correction de ce pH, une solution présentant un pH allant de 4 à 7 ;
- une étape d'hydrogénation dans un réacteur de ladite solution pendant une durée suffisante pour atteindre une composition, dite composition préhydrogénée, présentant une teneur en sucres réducteurs allant de 0,15 à 1 % ;
- suivie d'une étape de modification du pH de la composition préhydrogénée par ajout d'une base, ladite composition présentant à l'issue de cette étape un pH allant de 8,5 à 11 ;
- suivie d'une étape d'alcalinisation de la composition préhydrogénée, également en présence d'un catalyseur d'hydrogénation et d'hydrogène, à une température comprise entre 100 et 140°C pendant une durée suffisante pour former la composition de polymère de glucose hydrogénée.

Le procédé selon l'invention présente l'avantage de très peu dégrader la structure du polymère de glucose. Contrairement aux procédés de l'art antérieur (comme ceux décrits dans les documents US 5,424,418, EP 654483 ou WO 92/14761), le procédé de l'invention comprend une étape de modification du pH par ajout d'une base entre les deux étapes diminuant la quantité en sucres réducteurs du polymère de glucose, c'est-à-dire les étapes d'hydrogénation et d'alcalinisation. Comme démontré dans la partie exemples, ce sont ces deux étapes, réalisées à des pH distincts et dans cet ordre, qui permettent d'obtenir la composition de l'invention. Ceci est d'autant plus surprenant qu'il est bien connu que les polymères de glucose sont peu stables au pH de l'étape d'alcanisation. Sans être liée par une quelconque théorie, il semble que l'étape d'hydrogénation permette de rendre le polymère de glucose suffisamment stable pour pouvoir le soumettre de manière efficace à l'étape subséquente d'alcanisation et ainsi réduire drastiquement la quantité de sucres réducteurs dans la composition de l'invention.

L'invention va maintenant être décrite en détail ci-après.

## Description détaillée de l'invention

Un objet de l'invention porte sur une composition de polymère de glucose hydrogénée contenant des fibres alimentaires et présentant une quantité de sucres réducteurs SR très faible.

Par « composition de polymère de glucose hydrogénée », on entend une composition comprenant des polymères de glucose dont les fonctions terminales ont été hydrogénées.

Le polymère de glucose est un polymère de glucose contenant des fibres alimentaires non digestibles. La composition selon l'invention contient une teneur totale de fibres, déterminée selon la méthode AOAC 2001.03, supérieure à 50%, avantageusement supérieure à 60%, de préférence supérieure à 70%, par exemple supérieure à 80%.

Il existe de nombreux polymères de glucose contenant des fibres alimentaires qui ont été décrits dans la littérature. On peut par exemple citer les polymères de glucose contenant des fibres alimentaires obtenus par catalyse acide à partir de solutions aqueuses concentrées de glucose ou de sirops de glucose telles que ceux décrits dans les documents US 3876794 ou WO 9841545. Les polymères de glucose contenant des fibres alimentaires peuvent également être obtenus à partir d'amidon, tel que décrit par exemple dans les documents EP 535627 ou EP 538146 : on parle alors de dextrines indigestibles.

Selon une première variante préférée de l'invention, le polymère de glucose est le polymère de glucose décrit dans le document EP 1006128 au nom de la Demanderesse, c'est-à-dire une maltodextrine branchée, caractérisée par le fait qu'elle présente entre 22 % et 35 %, de préférence entre 27 et 34 % de liaisons glucosidiques 1 -> 6, une teneur en sucres réducteurs inférieure à 20 %, un indice de polymolécularité inférieur à 5 et une masse moléculaire moyenne en nombre Mn au plus égale à 4500 g/mole.

Selon une seconde variante préférée de l'invention, le polymère de glucose est le polymère de glucose décrit dans le document FR 3 032 709 au nom de la Demanderesse, c'est-à-dire un malto-oligo-saccharide présentant une teneur en liaisons $\alpha$ 1-4 comprise entre 70 % et 80 du nombre total de liaisons osidiques 1-4.

Des polymères de glucose contenant des fibres alimentaires sont également disponibles dans le commerce, comme par exemple le Nutriose® commercialisé par la Demanderesse, le Litesse® commercialisé par la société Danisco, le Promitor® commercialisé par la société Tate and Lyle ou encore le Fibersol®2 commercialisé par la société Matsutani.

Lorsque le polymère de glucose contient des fibres alimentaires, il est essentiel de ne dégrader que peu la structure du polymère de glucose lors de l'hydrogénation, ceci en vue de conserver les liaisons atypiques les caractérisant et donc les propriétés particulières de digestibilité de ces produits. L'invention est donc particulièrement intéressante puisque le procédé de l'invention permet de ne pas ou peu dégrader la structure du polymère de glucose et de ne pas ou peu diminuer la teneur totale de fibres. De manière surprenante, ceci a été rendu possible par le procédé particulier de l'invention alors qu'il est connu qu'en conditions basiques, un polymère de glucose se dégrade, ce qui conduit dans le cas d'un polymère de glucose contenant des fibres alimentaires à une perte des avantages nutritionnels liés à ces fibres. Par « peu dégrader la structure du polymère de glucose », on entend réduire, lors du procédé de fabrication de la composition de l'invention, la masse moléculaire Mn du polymère de glucose dans des proportions ne dépassant pas 10% de sa masse moléculaire Mn initiale. Par « peu diminuer la teneur totale de fibres », on entend réduire, lors du procédé de fabrication de la composition de l'invention, la teneur totale de fibres du polymère de glucose, déterminée selon la méthode AOAC 2001.03, dans des proportions ne dépassant pas 3% : par exemple, pour un polymère de glucose contenant 80% de fibres, un procédé diminue peu la teneur totale en fibres tant que cette teneur dans la composition hydrogénée obtenue n'est pas inférieure à 77%.

La composition de polymère de glucose hydrogénée de l'invention présente une quantité de sucres réducteurs inférieure à 800 ppm. Cette quantité est déterminée par la méthode Nelson-Somogyi, bien connue de l'Homme du métier, cette méthode ayant fait l'objet de la publication, Somogyi M. (1952) J. Biol. Chem., 200 245. Dans le contexte de l'invention, il est nécessaire d'utiliser cette méthode Nelson-Somogyi pour déterminer la quantité de sucres réducteurs dans la composition. En effet, les autres méthodes connues, par exemple la méthode Bertrand, ne permettent pas de déterminer de manière précise la quantité de sucres réducteurs lorsque celle-ci est aussi faible que celle que des compositions de l'invention. De préférence, la quantité de sucres réducteurs est inférieure à 700 ppm, par exemple inférieure à 600 ppm, par exemple inférieure à 500 ppm, par exemple inférieure à 400 ppm. La composition selon l'invention peut présenter une quantité de sucres réducteurs en poids sec qui va de 20 à 600 ppm, par exemple de 50 à 400 ppm.

La composition selon l'invention peut présenter, par rapport au poids sec de la composition, un poids sec en polyols (DP1H+DP2H) issus de l'hydrogénation du glucose, du maltose et de leurs isomères inférieur à 30%, avantageusement inférieur à 25%, par exemple inférieur à 15%, notamment inférieur à 5%, voire inférieur à 2%. Cette quantité peut être déterminée par chromatographie liquide haute performance.

Selon l'invention, la masse moléculaire moyenne en nombre Mn de la composition peut largement varier et notamment aller de 500 à 3000 g/mol, avantageusement de 800 à 2600 g/mol. Elle peut par exemple aller de 500 à 1600 g/mol ou de 1600 à 2300 g/mol. La masse moléculaire moyenne en poids Mw de la composition peut largement varier et notamment aller de 1000 à 6000 g/mol, avantageusement de 1300 à 5000 g/mol. Elle peut par exemple aller de 1000 à 3600 g/mol ou de 3600 à 6000 g/mol. On considère généralement que la masse moléculaire du polymère de glucose est élevée lorsque sa masse moléculaire moyenne en poids est supérieure ou égale à 2500 g/mol. Les masses moléculaires Mn et Mw dépendent essentiellement des masses moléculaires Mn et Mw du polymère de glucose soumis au procédé de l'invention. La masse moléculaire moyenne en poids peut être considérée comme un facteur prépondérant en ce qui concerne la viscosité du polymère de glucose. Dans la pratique, les valeurs de Mn et de Mw sont déterminées par une méthode de mesure adaptée aux polymères de glucose, qui peut reposer sur la chromatographie de perméation de gel sur des colonnes de chromatographie étalonnées avec des pullulans de masses moléculaires connues. La quantité SR dépend quant à elle essentiellement du polymère de glucose de départ, des conditions d'hydrogénation et des conditions d'alcalinisation qui vont être décrites ci-après. La composition selon l'invention peut présenter un ratio SR/Mw, SR étant exprimée en ppm et Mw étant exprimé en g/mol, allant de 0,01 à 0,80, notamment allant de 0,02 à 0,60, par exemple allant de 0,04 à 0,30.

Un objet de l'invention porte sur une composition de polymère de glucose hydrogénée présentant une masse moléculaire moyenne en poids supérieure ou égale à 2500 g/mol, par exemple allant de 2500 à 6000 g/mol, avantageusement allant de 3600 à 6000 g/mol, voire de 3700 à 5000 g/mol et une quantité SR allant de 50 à 800 ppm, par exemple de 80 à 600 ppm, voire de 100 à 400 ppm. Cette composition est particulièrement intéressante dans la mesure où le procédé de l'invention permet de diminuer drastiquement la quantité de sucres réducteurs, et ceci même dans cette variante où la masse moléculaire du polymère de glucose est élevée.

Un autre objet de l'invention porte sur une composition de polymère de glucose hydrogénée présentant une masse moléculaire moyenne en poids allant de 1000 à 3600 g/mol et une quantité SR allant de 30 à 800 ppm, par exemple de 40 à 500 ppm, voire de 50 à 250 ppm.

Les compositions de l'invention présentent une stabilité améliorée, et ceci dans une large gamme de pH allant d'acide à neutre. Ceci est un avantage dans la mesure où la très grande majorité des produits alimentaires présentent un tel pH. Ces stabilités améliorées permettent aux compositions de polymère de glucose d'être avantageusement utilisées dans ces produits alimentaires. Le détail des traitements est explicité dans la partie Exemples.

Ainsi, selon l'invention, la composition de polymère de glucose hydrogénée de l'invention a une stabilité améliorée à pH acide et présente avantageusement, après traitement thermique du TEST A, une coloration ICUMSA inférieure à 100, préférentiellement inférieure à 90, voire inférieure à 70. Selon l'invention, la composition de polymère de glucose hydrogénée de l'invention a une stabilité améliorée à pH neutre et présente avantageusement, après traitement thermique du TEST B, une coloration ICUMSA inférieure à 100, préférentiellement inférieure à 90, voire inférieure à 70.

Le traitement du TEST A consiste à mettre la composition de polymère de glucose hydrogénée sous la forme d'une composition aqueuse tamponnée à pH=4 et à 25% de matière sèche puis à effectuer un traitement thermique de cette composition aqueuse tamponnée pendant 6 heures à 115°C. Le traitement du TEST B consiste à mettre la composition de polymère de glucose hydrogénée sous la forme d'une composition aqueuse tamponnée à pH=7 et à 25% de matière sèche puis à effectuer un traitement thermique de cette composition aqueuse tamponnée pendant 6 heures à 85°C.

La composition selon l'invention a pu être obtenue par un procédé de fabrication comprenant :

- une étape de fourniture d'une solution aqueuse de polymère de glucose contenant des fibres alimentaires ;
- une étape de mise en contact de la solution aqueuse avec un catalyseur d'hydrogénation pour fournir, éventuellement après correction de ce pH, une solution présentant un pH allant de 4 à 7 ;
- une étape d'hydrogénation dans un réacteur de ladite solution pendant une durée suffisante pour atteindre une composition, dite composition préhydrogénée, présentant une teneur en sucres réducteurs allant de 0,15 à 1 % ;
- suivie d'une étape de modification du pH de la composition préhydrogénée par ajout d'une base, ladite composition présentant à l'issue de cette étape un pH allant de 8,5 à 11 ;
- suivie d'une étape d'alcalinisation de la composition préhydrogénée, également en présence d'un catalyseur d'hydrogénation et d'hydrogène, à une température comprise entre 100 et 140°C pendant une durée suffisante pour former la composition polymère de glucose hydrogénée.

La solution aqueuse de polymère de glucose contenant des fibres alimentaires fournie peut présenter une matière sèche allant de 15 à 40%, par exemple de 20 à 30%. L'Homme du métier peut faire varier la matière sèche de la solution selon le polymère de glucose et/ou le réacteur utilisés. En ce qui concerne les polymères de glucose fournis, ils dépendent de la composition de polymère de glucose hydrogénée souhaitée, notamment en termes de Mn, Mw et de teneur en fibres. Ce polymère de glucose peut présenter, par rapport au poids sec de la composition, un poids sec en sucres (DP1+DP2), c'est-à-dire en glucose, maltose et leurs isomères, inférieure à 30%, avantageusement inférieure à 25%, par exemple inférieure à 15%, notamment inférieure à 5%, voire inférieure à 2%. Cette quantité peut être déterminée par chromatographie liquide haute performance. Les polymères peuvent être choisis parmi ceux décrits précédemment.

On peut préparer la solution de polymère de glucose par simple mélange dans les quantités désirées du polymère de glucose avec de l'eau. La solution aqueuse de polymère de glucose est mise en contact avec un catalyseur d'hydrogénation. Les catalyseurs d'hydrogénation sont connus en soi ; ce catalyseur peut être de type Nickel de Raney ou tout autre catalyseur d'hydrogénation des sucres pouvant convenir. L'Homme du métier saura ajuster les quantités de catalyseur à utiliser ; à titre d'exemple, on peut utiliser du Nickel de Raney dans des quantités pouvant aller de 2 à 10%, par rapport à la masse sèche du polymère de glucose, par exemple environ 5%.

La solution aqueuse de polymère de glucose présente, avant le début de l'étape d'hydrogénation un pH allant de 4 à 7. Ce pH peut être corrigé par ajout d'un acide ou d'une base.

La solution aqueuse de polymère de glucose est ensuite soumise à l'étape d'hydrogénation dans un réacteur.

Les réacteurs permettant l'hydrogénation sont des réacteurs connus, capables de fonctionner sous pression ; il peut par exemple s'agir de réacteurs à double enveloppe. Le réacteur est généralement équipé d'un agitateur permettant l'homogénéisation du milieu réactionnel.

De préférence, la température lors de l'étape d'hydrogénation est comprise entre 100 et 140°C, tout préférentiellement entre 110 et 130°C. Avantageusement, la pression d'hydrogène dans le réacteur va de 20 à 200 bar, par exemple de 30 à 100 bar, préférentiellement de 40 à 60 bar.

A l'issue de cette étape est obtenue une composition, dite composition préhydrogénée, qui peut présenter une teneur en sucres réducteurs, déterminée par la méthode Nelson-Somogyi, allant de 0,15 à 1 %. Celle-ci est obtenue en réalisant l'étape d'hydrogénation pendant une durée suffisante pour obtenir cette teneur en sucres réducteurs. Cette durée varie principalement selon les conditions de pression et de température utilisées lors de cette étape : plus la pression et la température sont élevées, plus le temps de réaction peut être raccourci. Toutefois, l'Homme du métier préfère utiliser les conditions préférées décrites précédemment, ceci en vue de ne très peu dégrader la structure du polymère de glucose. La durée de cette étape peut aller de 90 à 210 minutes, de préférence de 120 à 180 minutes.

Cette composition préhydrogénée peut ensuite être soumise à une étape de modification de pH par ajout d'une base, ladite composition présentant à l'issue de cette étape un pH allant de 8,5 à 11, par exemple de 8,6 à 10, préférentiellement de 9,10 à 9,90. La base peut être de la soude.

Une étape d'alcalinisation de cette composition préhydrogénée est ensuite réalisée à une température comprise entre 100 et 140°C, et ceci pendant une durée suffisante pour former la composition polymère de glucose hydrogénée de l'invention.

La durée de l'étape d'alcalinisation varie principalement selon les conditions de température utilisées lors de cette étape : plus la température est élevée et la teneur en sucres réducteur de la composition préhydrogénée est basse, plus le temps de réaction peut être raccourci. Toutefois, dans les conditions indiquées, il est préférable d'utiliser une durée allant de 10 à 120 minutes et de préférence de 15 à 100 minutes pour ne dégrader que peu la structure du polymère de glucose. De manière surprenante, cette étape d'alcalinisation du procédé ne dégrade pas ou peu la structure et ne diminue pas ou peu la teneur totale de fibres du polymère de glucose ; sans être liée à une quelconque théorie, la Demanderesse fait l'hypothèse que la composition préhydrogénée fournie lors de l'étape d'hydrogénation présente des propriétés lui permettant de pouvoir être soumise à l'étape d'alcalinisation, et ceci sans dégrader la structure du polymère de glucose. Ceci est particulièrement essentiel lorsque le polymère de glucose comprend des fibres alimentaires et que l'on souhaite conserver les avantages nutritionnels de telles fibres.

L'étape d'alcalinisation se fait également en présence d'un catalyseur d'hydrogénation et d'hydrogène. La pression d'hydrogène peut aller de 20 à 200 bar, par exemple de 30 à 100 bar, préférentiellement de 40 à 60 bar. L'étape d'alcalinisation peut être conduite dans le réacteur d'hydrogénation décrit précédemment, sous hydrogène et sans séparation du catalyseur utilisé lors de l'étape d'hydrogénation, par introduction de la base à l'issue de cette dernière.

L'Homme du métier pourra faire varier les paramètres de l'étape d'alcalinisation décrits ci-dessus afin de diminuer la quantité de sucres réducteurs dans la composition hydrogénée de l'invention, par exemple en augmentant la température ou en augmentant la durée de cette étape, voire en augmentant la quantité de catalyseur et en augmentant la pression d'hydrogène.

Les étapes d'hydrogénation et d'alcalinisation peuvent être de type batch ou continu.

Il est possible de soutirer la composition préhydrogénée du réacteur, en vue de réaliser l'étape suivante d'alcalinisation dans un autre réacteur. Il est également possible de maintenir la composition dans le même réacteur, de réaliser l'étape de modification de pH dans celui-ci puis de réaliser l'étape d'alcalinisation.

Le catalyseur utilisé lors la première étape ou éventuellement lors de la seconde étape peut être séparé de la composition préhydrogénée ou de la composition de polymère de glucose hydrogénée par une étape de filtration, réalisée éventuellement après une étape de décantation.

Le procédé de l'invention peut également comprendre, à la suite de l'étape d'alcalinisation, une étape de purification de la composition de polymère de glucose hydrogénée. Avantageusement, l'étape de purification comprend un stade de passage de la composition de polymère de glucose hydrogénée sur une ou plusieurs résines échangeuses d'ions. Préférentiellement, ce stade comprend un premier passage sur une résine cationique forte puis un deuxième passage sur une résine anionique faible. Ce stade peut également être complété par un passage sur lit mixte. L'étape de purification peut également comprendre un traitement au noir de carbone de la composition de polymère de glucose.

Le procédé peut également comprendre une étape de chromatographie, par exemple une étape de chromatographie à lit mobile simulé.

La composition, encore sous forme liquide à ce stade, peut être mise sous forme solide. Avantageusement, la composition se présente sous la forme d'une poudre qui est de préférence une poudre atomisée. Le procédé peut ainsi comprendre une étape de concentration suivie d'une étape de séchage. L'étape de concentration peut se faire en utilisant tout type évaporateur et l'étape de séchage peut être notamment une étape d'atomisation ou une étape de granulation. Ces méthodes sont bien connues de l'Homme du métier.

La composition de polymère de glucose hydrogénée de l'invention peut être utilisée dans les applications déjà connues des polymères de glucose hydrogénés.

La composition selon l'invention peut notamment être utilisée pour la fabrication de produits alimentaires et pharmaceutiques et notamment pour la fabrication de boissons, telles que les boissons sans alcool, comme les boissons gazeuses et notamment les sodas, les boissons à base de thé, les jus de fruits, les boissons sans sucres ou réduites en sucres, ainsi que les boissons alcoolisées telles que les bières, les liqueurs et les spiritueux pour la fabrication des produits laitiers tels que les yaourts ou les crèmes glacées, pour la fabrication des produits de cuisson tels que le pain, les céréales, les gâteaux ou les biscuits, pour la fabrication des confiseries, pour la fabrication des confitures ou préparation à base de fruits, des sauces, pour la fabrication des pâtes ou des nouilles, pour la fabrication des aliments de nutrition particulière tels que les aliments pour personnes âgées ou jeunes enfants et nourrissons ainsi que les compléments alimentaires pouvant être sous forme de poudre ou de comprimés ou encore pour la fabrication des préparations pharmaceutiques.

Un autre objet de l'invention porte sur l'utilisation de la composition selon l'invention pour la fabrication de produits alimentaires et pharmaceutiques présentant un pH allant de 2 à 7.

L'invention va maintenant être détaillée dans les exemples suivants. Il est précisé que ces exemples ne sont pas limitatifs et ne sont en aucun cas susceptibles de limiter la portée de la présente invention.

## **Exemples**

MESURE DE LA COLORATION

La coloration est mesurée selon la méthode ICUMSA

Après filtration sur filtre à membrane de porosité 0,45 $\mu$m (filtre Acrodisc), on mesure la densité optique (DO) à 420 nm de la solution à l'aide d'un spectrophotomètre dans une cuve de 5 cm de longueur. On mesure également le brix de la solution

$$ICUMSA = \frac{DO * 100000}{Brix * Longueur\ de\ cuve(cm) * densité}$$

La coloration des différentes compositions de polymère de glucose hydrogénées est reportée dans les Tableaux 1 et 2 à la ligne « ICUMSA ».

METHODE DE DETERMINATION DE LA STABILITE

On réalise un test de stabilité des compositions de polymère de glucose hydrogénées à différents pH et températures.

L'objectif est d'évaluer l'évolution de la coloration pendant un temps donné. Ceci permet de caractériser la stabilité des fibres hydrogénées au cours d'un process alimentaire.

Pour ceci, on prépare deux solutions de fibres hydrogénées à 25 % de matière sèche.

Pour la détermination de la stabilité à pH 4 (TEST A), la solution est tamponnée à pH=4 (solution tampon Fluka® pH 4 comprenant acide citrique, hydroxyde de sodium et chlorure de sodium). Pour la détermination de la stabilité à pH 7 (TEST B), la solution est tamponnée à pH = 7 (solution tampon Fluka® pH 7 comprenant dihydrogéno-phosphate de potassium et hydrogénophosphate de sodium).

Les solutions sont ensuite placées dans des tubes à essais en pyrex fermés (10 tubes pour chaque pH) et les tubes sont placés dans un bain d'huile pendant 6 heures :

- à 115°C pour le TEST A qui étudie la stabilité à pH=4 ;
- et à 85%) pour le TEST B qui étudie la stabilité à pH=7.

Les tubes sont ensuite sortis du bain d'huile et refroidis à température ambiante (20°C).

De la même manière que précédemment, la coloration selon la méthode ICUMSA est mesurée pour les différents tubes à essais à la suite des traitements thermiques et la moyenne des mesures est réalisée.

Les colorations des différentes compositions de polymère de glucose hydrogénée après traitement sont reportées dans les Tableaux 1 et 2 aux lignes « ICUMSA (pH4, 6h) » et « ICUMSA (pH7, 6h) ».

EXEMPLE 1 A

Dans un réacteur à double enveloppe d'une capacité de 20 litres, contenant du Nickel de Raney en suspension, on introduit sous agitation un polymère de glucose de type Nutriose® FM 10.

Le Nutriose® FM 10 utilisé comprend environ 10% de sucres réducteurs, comprend un taux de fibres de 74% environ (AOAC 2001.03), présente une masse molaire moyenne en nombre de 1145 g/mol ainsi qu'une masse molaire moyenne en poids de 3480 g/mol.

La matière sèche en polymère de glucose dans le milieu réactionnel est de 25% en poids, et la teneur en Nickel de Raney est de 5% en poids, exprimés par rapport au poids sec. Le pH de la solution est égal à 5.

L'hydrogénation est conduite pendant 2h à une pression de 50 bars d'hydrogène et une température de 120°C.

On obtient une composition préhydrogénée comprenant une quantité de sucres réducteurs, déterminée selon la méthode Nelson-Somogyi de 1867 ppm.

On introduit une solution de soude à 3% en poids en 15 minutes de manière à amener le pH de la composition préhydrogénée à une valeur de 9,2, tout en maintenant le réacteur à une pression de 50 bars d'hydrogène et une température de 120°C. On réalise ensuite l'étape d'alcalinisation pendant 15 minutes.

Après quoi, on arrête la réaction et laisse décanter pendant 15 minutes et on vidange le surnageant vers un décanteur afin de récupérer le catalyseur. Le surnageant du décanteur est ensuite filtré afin d'éliminer les dernières traces de catalyseur.

On soumet le sirop ainsi obtenu, après l'avoir refroidi à 15°C, à une purification sur résine cationique forte et anionique faible, puis sur lit mixte.

Les caractéristiques de la composition hydrogénée se retrouvent dans le Tableau 1 ci-dessous.

EXEMPLE 1 B

L'Exemple 1B diffère de l'exemple 1A uniquement en ce que la durée de l'étape d'alcanisation est de 45 minutes au lieu de 15 minutes.

Les caractéristiques de la composition hydrogénée se retrouvent dans le Tableau 1 ci-dessous.

EXEMPLE 1 C

L'Exemple 1C diffère de l'exemple 1A uniquement en ce que la durée de l'étape d'alcanisation est de 75 minutes au lieu de 15 minutes.

Les caractéristiques de la composition hydrogénée se retrouvent dans le Tableau 1 ci-dessous.

EXEMPLE COMPARATIF 1

L'Exemple comparatif 1 (C EX1) diffère de l'exemple 1A uniquement en ce que la durée de l'étape d'hydrogénation dure 3h et qu'aucune étape d'alcanisation n'est réalisée.

Les caractéristiques de la composition hydrogénée se retrouvent dans le Tableau 2 ci-dessous.

EXEMPLE 2 A

Dans un réacteur à double enveloppe d'une capacité de 20 litres, contenant du Nickel de Raney en suspension, on introduit sous agitation un polymère de glucose de type Nutriose® FM06.

Le Nutriose® FM 06 utilisé comprend environ 5% de sucres réducteurs, un taux de fibres de 85% environ (AOAC 2001.03), présente une masse molaire moyenne en nombre de 2295 g/mol ainsi qu'une masse molaire moyenne en poids de 4125 g/mol.

La matière sèche en polymère de glucose dans le milieu réactionnel est de 25% en poids, et la teneur en Nickel de Raney est de 5% en poids, exprimés par rapport au poids sec. Le pH de la solution est égal à 5.

L'hydrogénation est conduite pendant 3h à une pression de 50 bars d'hydrogène et une température de 130°C

On obtient une composition préhydrogénée comprenant une quantité de sucres réducteurs, déterminée selon la méthode Nelson-Somogyi de 1673 ppm.

On introduit une solution de soude à 3% en poids en 15 minutes de manière à amener le pH de la composition préhydrogénée à une valeur de 9,3, tout en maintenant le réacteur à une pression de 50 bars d'hydrogène et une température de 120°C. On réalise ensuite l'étape d'alcalinisation pendant 15 minutes.

Après quoi, on arrête la réaction et laisse décanter pendant 15 minutes et on vidange le surnageant vers un décanteur afin de récupérer le catalyseur. Le surnageant du décanteur est ensuite filtré afin d'éliminer les dernières traces de catalyseur.

On soumet le sirop ainsi obtenu, après l'avoir refroidi à 15°C, à une purification sur résine cationique forte et anionique faible, puis sur lit mixte.

Les caractéristiques de la composition hydrogénée se retrouvent dans le Tableau 1 ci-dessous.

EXEMPLE 2 B

L'Exemple 2B diffère de l'exemple 2A uniquement en ce que la durée de l'étape d'hydrogénation est de 2h au lieu de 3h et en ce que la durée de l'étape l'alcanisation est de 75 minutes au lieu de 15 minutes.

On obtient une composition préhydrogénée comprenant une quantité de sucres réducteurs, déterminée selon la méthode Nelson-Somogyi de 3255 ppm. Après introduction de la soude, le pH de la composition préhydrogénée a une valeur de 9,6.

Les caractéristiques de la composition hydrogénée se retrouvent dans le Tableau 1 ci-dessous.

EXEMPLE 2 C

L'Exemple 2C diffère de l'exemple 2B uniquement en ce que la teneur en Nickel de Raney est de 7,5% en poids, exprimés par rapport au poids sec, au lieu de 5%.

On obtient une composition préhydrogénée comprenant une quantité de sucres réducteurs, déterminée selon la méthode Nelson-Somogyi de 2163 ppm. Après introduction de la soude, le pH de la composition préhydrogénée a une valeur de 9,7.

Les caractéristiques de la composition hydrogénée se retrouvent dans le Tableau 1 ci-dessous.

EXEMPLE COMPARATIF 2A

L'Exemple comparatif 2 A (C EX2 A) diffère de l'exemple 2C uniquement en ce que la durée de l'étape d'hydrogénation dure 3h et qu'aucune étape d'alcanisation n'est réalisée.

Les caractéristiques de la composition hydrogénée se retrouvent dans le Tableau 2 ci-dessous.

EXEMPLE COMPARATIF 2B

L'Exemple comparatif 2B (C EX2 B) diffère de l'exemple 2C uniquement en ce que la durée de l'étape d'hydrogénation dure 4h et qu'aucune étape d'alcanisation n'est réalisée.

Les caractéristiques de la composition hydrogénée se retrouvent dans le Tableau 2 ci-dessous.

EXEMPLE 3

On fournit une composition de polymère de glucose en reproduisant l'exemple 1 de la demande FR 3 032 709 A1.

La composition fabriquée comprend environ 20,2% de sucres réducteurs, un taux de fibres de 61% environ (AOAC 2001.03), présente une masse molaire moyenne en nombre de 595 g/mol ainsi qu'une masse molaire moyenne en poids de 1300 g/mol.

La matière sèche en polymère de glucose dans le milieu réactionnel est de 25% en poids, et la teneur en Nickel de Raney est de 5% en poids, exprimés par rapport au poids sec. Le pH de la solution est égal à 5.

L'hydrogénation est conduite pendant 2h à une pression de 50 bars d'hydrogène et une température de 130°C

On obtient une composition préhydrogénée comprenant une quantité de sucres réducteurs, déterminée selon la méthode Nelson-Somogyi de 1570 ppm.

On introduit une solution de soude à 3% en poids en 15 minutes de manière à amener le pH de la composition préhydrogénée à une valeur de 9,3, tout en maintenant le réacteur à une pression de 50 bars d'hydrogène et une température de 120°C. On réalise ensuite l'étape d'alcalinisation pendant 75 minutes.

Après quoi, on arrête la réaction et laisse décanter pendant 15 minutes et on vidange le surnageant vers un décanteur afin de récupérer le catalyseur. Le surnageant du décanteur est ensuite filtré afin d'éliminer les dernières traces de catalyseur.

On soumet le sirop ainsi obtenu, après l'avoir refroidi à 15°C, à une purification sur résine cationique forte et anionique faible, puis sur lit mixte.

Les caractéristiques de la composition hydrogénée se retrouvent dans le Tableau 2 ci-dessous.

Dans le Tableau 1 figure également les caractéristiques de 2 compositions de polymères de glucose hydrogénées commerciales, Litesse® Ultra qui est un polydextrose hydrogéné commercialisé par la société Danisco ou Fibersol® 2H qui est une dextrine indigestible commercialisée par la société Matsutani.

Tableau 1 : Caractérisation des compositions de polymère de glucose hydrogénées

|  | Ex 1A | Ex 1B | Ex 1C | Ex 2A | Ex 2B | Ex 2C | Fibersol® 2H | Litesse® Ultra |
|---|---|---|---|---|---|---|---|---|
| SR | 614 ppm | 370 ppm | 86 ppm | 714 ppm | 391 ppm | 208 ppm | 2501 ppm | 1503 ppm |
| DP1H+DP2H | 4,0 | 4,0 | 4,0 | 0,3 | 0,2 | 0,3 | n.d. | n.d. |
| Mn (g/mol) | 1170 | 1170 | 1170 | 2210 | 2150 | 2170 | 1245 | 715 |
| Mw (g/mol) | 3465 | 3465 | 3415 | 4030 | 3995 | 4000 | 2810 | 1600 |
| Fibres totales | 70% | n.d. | 69% | 81% | 80% | 80% | n.d. | 79% |
| ICUMSA | 22 | 15 | 6 | 25 | 18 | 14 | 29 | 28 |
| ICUMSA (pH4, 6h) | 62 | 44 | 32 | 87 | 56 | 38 | 154 | 128 |
| ICUMSA (pH7, 6h) | 85 | 53 | 41 | 96 | 58 | 47 | 184 | 140 |
| n.d. : non déterminé | | | | | | | | |

Tableau 2 : Caractérisation des compositions de polymère de glucose hydrogénées

|  | Ex 3 | C Ex1 | C Ex2A | C Ex2B |
|---|---|---|---|---|
| SR | 320 ppm | 1620 ppm | 1150 ppm | 1080 ppm |
| DP1 H+DP2H | 20,2 | 4,0 | 0,3 | 0,3 |
| Mn (g/mol) | 595 | 1170 | 2210 | 2210 |
| Mw (g/mol) | 1300 | 3465 | 4030 | 4030 |
| Fibres totales | 61% | 70% | 81% | 81% |
| ICUMSA | 25 | 30 | 28 | 28 |
| ICUMSA (pH4, 6h) | 29 | 125 | 112 | 105 |
| ICUMSA (pH7, 6h) | 39 | 148 | 123 | 116 |

Ces exemples démontrent la possibilité de réaliser des compositions de polymère de glucose hydrogénées présentant une quantité de sucres réducteurs très faible, bien inférieure à celles des compositions de l'art antérieur.

La comparaison entre la composition de l'exemple comparatif 1 et la composition préhydrogénée de l'Exemple 1A démontre que la quantité de sucres réducteurs n'est pas réduite de manière significative, ceci bien que la durée soit augmentée d'une heure.

La comparaison entre la composition de l'exemple comparatif 2A avec la composition préhydrogénée de l'Exemple 2C démontre que la quantité de sucres réducteurs, bien que réduite, ne l'est pas suffisamment pour atteindre la quantité de sucres réducteurs de l'invention, ceci bien que la durée soit augmentée d'une heure.

La comparaison entre la composition de l'exemple comparatif 2B avec la composition de l'exemple comparatif 2A démontre que la quantité de sucres réducteurs n'est pas réduite de manière significative, ceci bien que la durée soit encore augmentée d'une heure.

Ainsi, quelles que soient les conditions sélectionnées, seul le procédé spécifique de l'invention, comprenant une étape d'hydrogénation et une étape d'alcalinisation, a permis d'obtenir une composition de polymère de glucose hydrogénée présentant une quantité de sucres réducteurs très faible, inférieure à 800 ppm.

Les essais démontrent également que les compositions selon l'invention présentent une stabilité thermique améliorée, à pH acide ou neutre. Sans être liée par une quelconque théorie, cela peut s'expliquer par la diminution drastique des sucres réducteurs.

Ceci est un avantage particulièrement recherché pour un produit qui pourra être utilisé dans des procédés de fabrication de produits alimentaires, un produit alimentaire présentant très généralement un pH allant de 2 à 7.

## Revendications

1. Composition de polymère de glucose hydrogénée présentant une teneur totale de fibres, déterminée selon la méthode AOAC 2001.03, supérieure à 50% et une quantité de sucres réducteurs en poids sec SR inférieure à 800 ppm.

2. Composition selon la revendication précédente, **caractérisée en ce que** sa quantité de sucres réducteurs en poids sec va de 20 à 600 ppm, de préférence de 50 à 400 ppm.

3. Composition selon l'une des revendications précédentes, **caractérisée en ce que** le polymère de glucose est un polymère de glucose contenant des fibres alimentaires non digestibles.

4. Composition selon la revendication 3, **caractérisée en ce que** sa teneur totale de fibres, déterminée selon la méthode AOAC 2001.03 est supérieure à 60%, de préférence supérieure à 70%, par exemple supérieure à 80%.

5. Composition selon l'une des revendications précédentes, **caractérisée en ce que**, par rapport au poids sec de la composition, le poids sec en polyols (DP1H+DP2H) issus de l'hydrogénation du glucose, du maltose et de leurs isomères est inférieure à 30%, avantageusement inférieure à 25%, par exemple inférieure à 15%, notamment inférieure à 5%, voire inférieure à 2%.

6. Composition selon l'une des revendications précédentes, **caractérisée en ce qu'**elle présente une Mw allant de 1000 à 6000 g/mol, avantageusement de 1300 à 5000 g/mol, par exemple allant de 1000 à 3600 g/mol ou de 3600 à 6000 g/mol.

7. Composition selon l'une des revendications 1 à 5, **caractérisée en ce qu'**elle présente une Mw supérieure ou égale à 2500 g/mol, par exemple allant de 2500 à 6000 g/mol.

8. Procédé de fabrication d'une composition de polymère de glucose hydrogénée selon l'une des revendications précédentes, comprenant :

   • une étape de fourniture d'une solution aqueuse de polymère de glucose contenant des fibres alimentaires ;
   • une étape de mise en contact de la solution aqueuse avec un catalyseur d'hydrogénation pour fournir, éventuellement après correction de ce pH, une solution présentant un pH allant de 4 à 7 ;
   • une étape d'hydrogénation dans un réacteur de ladite solution pendant une durée suffisante pour atteindre une composition, dite composition préhydrogénée, présentant une teneur en sucres réducteurs allant de 0,15 à 1 % ;
   • suivie d'une étape de modification du pH de la composition préhydrogénée par ajout d'une base, ladite composition présentant à l'issue de cette étape un pH allant de 8,5 à 11 ;
   • suivie d'une étape d'alcalinisation, également en présence d'un catalyseur d'hydrogénation et d'hydrogène, de la composition préhydrogénée à une température comprise entre 100 et 140°C pendant une durée suffisante pour former la composition polymère de glucose hydrogénée.

9. Procédé selon la revendication 8, **caractérisé en ce que** l'étape d'hydrogénation est réalisée à une température comprise entre 100 et 140°C et une pression d'hydrogène comprise entre 20 et 200 bar.

10. Procédé selon l'une des revendications 8 et 9, **caractérisé en ce que** la composition préhydrogénée présente un pH, à l'issue de l'étape de modification du pH, allant de 9,10 à 9,90.

11. Procédé selon l'une des revendications 8 à 10, **caractérisé en ce qu'**est réalisée, à la suite de l'étape d'alcalinisation, une étape de purification de la composition de polymère de glucose hydrogénée.

12. Procédé selon la revendication 11 **caractérisé en ce que** l'étape de purification comprend un stade de passage de la composition de polymère de glucose hydrogénée sur une ou plusieurs résines échangeuses d'ions.

**13.** Procédé selon l'une ou l'autre des revendications 11 et 12, **caractérisé en ce que** l'étape de purification comprend un traitement au noir de carbone de la composition de polymère de glucose.

**14.** Procédé selon l'une des revendications 8 à 13, **caractérisé en ce qu'**il comprend en outre une étape de concentration suivie d'une étape de séchage.

**15.** Produit alimentaire ou pharmaceutique comprenant la composition selon l'une des revendications 1 à 7.

**Patentansprüche**

**1.** Hydrierte Glucosepolymerzusammensetzung mit einem Gesamtfasergehalt, bestimmt nach der Methode AOAC 2001.03, von mehr als 50 % und einer Menge an reduzierenden Zuckern im Trockengewicht SR von weniger als 800 ppm.

**2.** Zusammensetzung nach dem vorhergehenden Anspruch, **dadurch gekennzeichnet, dass** ihre Menge an reduzierenden Zuckern im Trockengewicht von 20 bis 600 ppm, bevorzugt von 50 bis 400 ppm reicht.

**3.** Zusammensetzung nach einem der vorhergehenden Ansprüche, **dadurch gekennzeichnet, dass** das Glukosepolymer ein Glukosepolymer ist, das unverdauliche Nahrungsfasern enthält.

**4.** Zusammensetzung nach Anspruch 3, **dadurch gekennzeichnet, dass** ihr Gesamtfasergehalt, bestimmt nach der Methode AOAC 2001.03, größer als 60 %, bevorzugt größer als 70 %, z. B. größer als 80 % ist.

**5.** Zusammensetzung nach einem der vorhergehenden Ansprüche, **dadurch gekennzeichnet, dass**, bezogen auf das Trockengewicht der Zusammensetzung, das Trockengewicht an Polyolen (DP1H+DP2H), die aus der Hydrierung von Glucose, Maltose und ihren Isomeren stammen, weniger als 30 %, bevorzugt weniger als 25 %, beispielsweise weniger als 15 %, insbesondere weniger als 5 %, sogar weniger als 2 %, beträgt.

**6.** Zusammensetzung nach einem der vorhergehenden Ansprüche, **dadurch gekennzeichnet, dass** sie ein Mw von 1000 bis 6000 g/mol, vorteilhafterweise von 1300 bis 5000 g/mol, beispielsweise von 1000 bis 3600 g/mol oder von 3600 bis 6000 g/mol aufweist.

**7.** Zusammensetzung nach einem der Ansprüche 1 bis 5, **dadurch gekennzeichnet, dass** sie ein Mw von größer oder gleich 2500 g/mol, beispielsweise von 2500 bis 6000 g/mol, aufweist.

**8.** Verfahren zur Herstellung einer hydrierten Glukosepolymerzusammensetzung nach einem der vorhergehenden Ansprüche, umfassend :

    • einen Schritt des Bereitstellens einer wässrigen Glukosepolymerlösung, die Nahrungsfasern enthält,
    • einen Schritt des Inkontaktbringens der wässrigen Lösung mit einem Hydrierungskatalysator, um, gegebenenfalls nach Korrektur dieses pH-Werts, eine Lösung mit einem pH-Wert im Bereich von 4 bis 7 bereitzustellen,
    • einen Schritt der Hydrierung der Lösung in einem Reaktor während einer ausreichenden Dauer, um eine Zusammensetzung zu erhalten, die als vorhydrierte Zusammensetzung bezeichnet wird und einen Gehalt an reduzierenden Zuckern von 0,15 bis 1 % aufweist,
    • gefolgt von einem Schritt der Änderung des pH-Werts der vorhydrierten Zusammensetzung durch Zugabe einer Base, wobei die Zusammensetzung nach diesem Schritt einen pH-Wert von 8,5 bis 11 aufweist,
    • gefolgt von einem Schritt der Alkalisierung, ebenfalls in Gegenwart eines Hydrierungskatalysators und von Wasserstoff, der vorhydrierten Zusammensetzung bei einer Temperatur zwischen 100 und 140 °C über einen Zeitraum, der ausreicht, um die hydrierte Glucosepolymerzusammensetzung zu bilden.

**9.** Verfahren nach Anspruch 8, **dadurch gekennzeichnet, dass** der Hydrierungsschritt bei einer Temperatur von 100 bis 140 °C und einem Wasserstoffdruck von 20 bis 200 bar durchgeführt wird.

**10.** Verfahren nach einem der Ansprüche 8 und 9, **dadurch gekennzeichnet, dass** die vorhydrierte Zusammensetzung nach dem Schritt der pH-Modifikation einen pH-Wert von 9,10 bis 9,90 aufweist.

**11.** Verfahren nach einem der Ansprüche 8 bis 10, **dadurch gekennzeichnet, dass** im Anschluss an den Alkalisierungsschritt ein Reinigungsschritt für die hydrierte Glucosepolymerzusammensetzung durchgeführt wird.

12. Verfahren nach Anspruch 11, **dadurch gekennzeichnet, dass** der Reinigungsschritt eine Stufe umfasst, bei der die hydrierte Glukosepolymerzusammensetzung über ein oder mehrere Ionenaustauscherharze geleitet wird.

13. Verfahren nach einem der Ansprüche 11 oder 12, **dadurch gekennzeichnet, dass** der Reinigungsschritt eine Rußbehandlung der Glucosepolymerzusammensetzung umfasst.

14. Verfahren nach einem der Ansprüche 8 bis 13, **dadurch gekennzeichnet, dass** es ferner einen Konzentrationsschritt gefolgt von einem Trocknungsschritt umfasst.

15. Lebensmittel- oder Arzneimittelprodukt, das die Zusammensetzung nach einem der Ansprüche 1 bis 7 enthält.

**Claims**

1. Hydrogenated glucose polymer composition having a total fibre content, determined in accordance with AOAC method 2001.03, greater than 50% and a quantity of reducing sugars by dry weight SR of less than 800 ppm.

2. Composition according to the preceding claim, **characterised in that** the quantity of reducing sugars by dry weight thereof ranges from 20 to 600 ppm, preferably from 50 to 400 ppm.

3. Composition according to one of the preceding claims, **characterised in that** the glucose polymer is a glucose polymer containing non-digestible dietary fibres.

4. Composition according to claim 3, **characterised in that** the total fibre content thereof, determined in accordance with AOAC method 2001.03, is greater than 60%, preferably greater than 70%, example greater than 80%.

5. Composition according to one of the preceding claims, **characterised in that**, with respect to the dry weight of the composition, the dry weight of polyols (DP1H+DP2H) resulting from the hydrogenation of the glucose, maltose and isomers thereof is less than 30%, advantageously less than 25%, for example less than 15%, in particular less than 5%, or even less than 2%.

6. Composition according to one of the preceding claims, **characterised in that** it has an Mw ranging from 1000 to 6000 g/mol, advantageously from 1300 to 5000 g/mol, example ranging from 1000 to 3600 g/mol or from 3600 to 6000 g/mol

7. Composition according to one of claims 1 to 5, **characterised in that** it has an Mw greater than or equal to 2500 g/mol, example ranging from 2500 to 6000 g/mol.

8. Method for manufacturing a hydrogenated glucose polymer composition according to one of the preceding claims, comprising:

   • a step of providing an aqueous glucose polymer solution containing dietary fibres;
   • a step of contacting the aqueous solution with a hydrogenation catalyst to provide, optionally after correcting this pH, a solution having a pH ranging from 4 to 7;
   • a step of hydrogenation of said solution in a reactor for a sufficient time to achieve a composition, referred to as a pre-hydrogenated composition, having a reducing sugar content ranging from 0.15 to 1%;
   • followed by a step of modifying the pH of the pre-hydrogenated composition by adding a base, said composition having a pH ranging from 8.5 to 11 at the end of this step;
   • followed by a step of alkalinisation, also in the presence of a hydrogenation catalyst and hydrogen, of the pre-hydrogenated composition at a temperature between 100 and 140°C for a sufficient time to form the hydrogenated glucose polymer composition.

9. Method according to claim 8, **characterised in that** the hydrogenation step is implemented at a temperature between 100 and 140°C and a hydrogen pressure of between 20 and 200 bar.

10. Method according to one of claims 8 and 9, **characterised in that** the pre-hydrogenated composition has a pH, at the end of the pH modification step, ranging from 9.10 to 9.90.

11. Method according to one of claims 8 to 10, **characterised in that**, following the alkalinisation step, a step of purifying the hydrogenated glucose polymer composition is implemented.

12. Method according to claim 11, **characterised in that** the purification step comprises a stage of passing the hydrogenated glucose polymer composition over one or more ion exchange resins.

13. Method according to one or other of claims 11 and 12, **characterised in that** the purification step comprises a treatment of the glucose polymer composition with carbon black.

14. Method according to one of claims 8 to 13, **characterised in that** it furthermore comprises a concentration step followed by a drying step.

15. Nutritional or pharmaceutical product comprising the composition according to one of claims 1 to 7.

## RÉFÉRENCES CITÉES DANS LA DESCRIPTION

**Documents brevets cités dans la description**

- WO 9936442 A
- EP 530111 A
- EP 485304 A
- EP 516491 A
- US 4622233 A
- WO 9214761 A
- US 5424418 A
- EP 654483 A
- EP 368451 A
- US 20030096055 A1
- EP 516491 A2
- FR 2668147 A1
- EP 0561088 A2
- US 3876794 A
- WO 9841545 A
- EP 535627 A
- EP 538146 A
- EP 1006128 A
- FR 3032709
- FR 3032709 A1

**Littérature non-brevet citée dans la description**

- **SOMOGYI M.** *J. Biol. Chem.,* 1952, 200-245